# EUROPÄISCHE PATENTSCHRIFT

(11) **EP 3 022 551 B1**
(45) Veröffentlichungstag und Bekanntmachung des Hinweises auf die Patenterteilung: **05.09.2018**
(21) Anmeldenummer: 14753212.1
(22) Anmeldetag: 14.07.2014
(51) Int. Cl.: G01N 27/404

(54) **GALVANISCHER SAUERSTOFFSENSOR FÜR DIE MESSUNG IN GASGEMISCHEN**
GALVANIC OXYGEN SENSOR FOR MEASUREMENT IN GAS MIXTURES
CAPTEUR GALVANIQUE POUR LA MESURE D'OXYGÈNE DANS DES MÉLANGES GAZEUX

(30) Priorität: 15.07.2013 DE 102013011773
(43) Veröffentlichungstag der Anmeldung: 25.05.2016
(73) Patentinhaber: It Dr. Gambert GmbH, 23966 Wismar (DE)
(72) Erfinder: KIRSCH, Uwe, 23968 Zierow (DE); WEX, Kerstin, 19069 Alt Meteln (DE)
(74) Vertreter: Vossius & Partner Patentanwälte Rechtsanwälte mbB
(86) Internationale Anmeldenummer: PCT/EP2014/065015
(87) Internationale Veröffentlichungsnummer: WO 2015/007675

(56) Entgegenhaltungen:
- WO-A1-99/22229
- DE-A1- 4 135 824
- DE-A1-102006 024 022
- GB-A- 1 255 353

## Beschreibung

Die Erfindung betrifft generell einen galvanischen Sauerstoffsensor zur Messung in Gasgemischen. Insbesondere betrifft die Erfindung einen Sauerstoffsensor der die Forderungen der europäischen RoHS Gesetzgebung (2002/95/EG(ROHS-I) bzw. Richtlinie 2011/65/EU (RoHS II) erfüllt, der in seiner elektrischen und/oder geometrischen Spezifikation vorzugsweise rückwärtskompatibel ist zu bestehenden bleihaltigen Sensoren. Zudem betrifft die Erfindung vorzugsweise einen Sauerstoffsensor der vorzugsweise keine Querempfindlichkeit gegenüber Lachgas aufweist.

### HINTERGRUND DER ERFINDUNG

Aufgrund ihrer Zuverlässigkeit und ihrer kleinen Baugröße, bei einem günstigen Preis-Leistungsverhältniss werden galvanische Sauerstoffsensoren in der industriellen Messtechnik, Umweltmesstechnik und Medizinmesstechnik in erheblichen Umfang eingesetzt. Ausgehend von ihrer Entwicklung in den 50er Jahren entstanden zur Messung des Sauerstoffpartialdrucks eine Vielzahl von Geräten und Messinstrumenten, die teilweise noch heute in Gebrauch sind und die diese Art von Sensoren einsetzen. Auch in modernen Geräten wie Beatmungsmaschinen, Emissionsmessgeräten und Automobilabgasmessgeräten werden derartige Sensoren heute noch bevorzugt zur Sauerstoffmessung eingesetzt.

Die Sensoren bestehen meist aus einem Gehäuse, einer Kathode, einer Anode mit einer größeren Oberfläche als die Kathode, einer Diffusionsbarriere, einem Elektrolyt und Kontaktdrähten zur elektrischen Kontaktierung von Kathode und Anode. Die Anode liefert das notwendige elektrochemische Potential für die Reduktion des Sauerstoffs an der Kathode. Galvanische Sensoren sind z.B. in den Patenten US 3,767,552 und US 3,429,796 beschrieben.

In der Praxis hat sich Blei als Anodenmaterial durchgesetzt. Es weist eine hohe Wasserstoffüberspannung auf und ist daher in alkalischen und leicht sauren Elektrolyten über einen weiten Temperaturbereich weitgehend korrosionsfest. Die relativ hohe Dichte des Bleis erlaubt kleine Bauformen der Anode. Zudem lässt es sich als weiches Metall leicht bearbeiten und steht in hoher Reinheit relativ preiswert zur Verfügung.

Seit einigen Jahren ist Blei neben anderen Schwermetallen wie Cadmium, Quecksilber und Chrom als Werkstoff zunehmend in Verruf geraten. Bereits in relativ kleinen Dosen übt Blei bei chronischer Einwirkung im menschlichen Organismus eine schädigende Wirkung auf das Nerven- und Blutbildungssystem sowie auf die Nieren aus. Die Grenzwerte für Blei und Bleiverbindungen in der Umwelt wurden daher in den vergangenen Jahren immer weiter reduziert.

Die europäische Richtlinie (RoHS 2002/95/EG, das Kürzel RoHS steht für "Restriction of the use of certain Hazardous Substances"), erlaubt seit dem 1. Juni 2006 nur noch minimale Spuren dieser Substanzen in elektrischen und elektronischen Geräten. Für Blei liegt die untere Grenze der noch erlaubten Menge bei 0,1 % des homogenen Materials. Diese Richtlinie wurde am 8. Juni 2011 aktualisiert. Die Richtlinie 2011/65/EU, auch RoHS-II genannt, weitet den Geltungsbereich für die Stoffverbote in mehreren Stufen aus. Ab 2019 gelten sie für alle elektrischen und elektronischen Geräte, die nicht ausdrücklich ausgenommen sind. Ab 22. Juli 2014 fallen auch medizinische Geräte und Überwachungs- und Kontrollgeräte unter diese Gesetzgebung.

Insbesondere unterliegen die folgenden Stoffe den Beschränkungen gemäß Artikel 4 Absatz 1, wobei zulässige Höchstkonzentrationen in homogenen Werkstoffen in Anhang II wie folgt in Gewichtsprozent angegeben werden: Blei (0,1 %); Quecksilber (0,1 %); Cadmium (0,01 %); Sechswertiges Chrom (0,1 %); Polybromierte Biphenyle (PBB) (0,1 %) und Polybromierte Diphenylether (PBDE) (0,1 %).

Diese Gesetzgebung hat bei Messgeräteherstellern und ihren Zulieferern auf breiter Front zur Entwicklung von konformen, d.h. quasi schwermetallfreien Produkten geführt. Da auch weitere Schwermetalle, wie z.B. Cadmium und Quecksilber unter diese "Schwermetallverordnungen" fallen, stellen diese Metalle keine Alternative für den Einsatz in elektrochemischen Sauerstoffsensoren dar. Die Hersteller solcher Sensoren haben daher unterschiedliche Ansätze zur Lösung dieser Aufgabe erarbeitet.

Amperometrische Sensoren sind für die Messung von Gasen wie z.B. Kohlenmonoxid, Stickoxiden und Schwefelwasserstoff weit verbreitet. Überwiegend werden in ihnen drei Elektroden eingesetzt. Dabei wird durch eine potentiostatische Schaltung, die fest mit dem Sensor verbunden wird, zwischen der Arbeits- und Referenzelektrode ein konstantes Potential aufrecht erhalten. Hierfür muss der Potentiostat durch eine Spannungsquelle im Messgerät oder über eine in die Potentiostatschaltung integrierte Batterie versorgt werden. Als Elektrodenmaterialien kommen typischerweise Kohlenstoff in Kombination mit Edelmetallen zum Einsatz. Daher erfüllen solche Sensoren die Forderungen der RoHS-Gesetzgebung. Sauerstoffsensoren gemäß dieses Prinzips bieten z.B. die Firma Membrapor unter der Bezeichnung O2/M-100 und die Firma City Technology unter der Bezeichnung 4OXeco LP an. Nachteilig an diesem Prinzip ist die erforderliche Spannungsversorgung des Sensors. Diese verhindert die Rückwärtskompatibiltät zu galvanischen Sauerstoffsensoren. Weiterhin nachteilig ist die Adaptionszeit von mehreren Stunden nach der Installation im Messgerät.

Die US 2008/0274401 beschreibt ebenfalls einen amperometrischen, RoHS konformen Sauerstoffsensor. In diesem Sensor wird mit Hilfe einer im Sensor integrierten Knopfzellbatterie mit Hilfe einer dritten Elektrode eine Bias-Spannung angelegt. Diese muss über die Lebenszeit des Sensors exakt aufrecht erhalten werden. Weiterhin muss konstruktiv sichergestellt sein, dass kein Sauerstoff an die Referenzelektrode gelangt, weil sonst die Biasspannung zusammenbricht und dann keine zuverlässige Messung mehr möglich ist. Die Konstruktion eines solchen Sensors erfordert durch die Batterie im Sensor,die dritte Elektrode und die notwendige Abschirmung der Referenzelektrode gegenüber Sauerstoff ein wesentlich aufwändigeres Design des Sensors und der Sensorplatine als in galvanischen Sauerstoffsensoren. Dies erhöht die Herstellkosten deutlich. Wie galvanische Sensoren hat dieser Sensor eine durch die Kapazität der Batterie begrenzte Lebenszeit. Diese kann aufgrund eines hohen Stromverbrauchs des Sensors sehr limitiert sein. Ein weiterer Nachteil im Vergleich zu galvanischen Sensoren kann die schlechtere elektromagnetische Verträglichkeit des Sensors bedingt durch die integrierte elektronische Schaltung sein. Dies kann zusätzliche Massnahmen zu Abschirmung des Sensors im Messgerät erfordern.

Neben den am weitesten verbreiteten Bleianoden in galvanischen Sauerstoffsensoren werden bereits seit geraumer Zeit Anodenmaterialien ohne giftige Schwermetalle eingesetzt.

In der Patentschrift GB 1 255 353 wird ein galvanischer Sauerstoffsensor beschrieben, dessen Anodenmaterial neben Blei aus Zinn, Kupfer und deren Legierungen bestehen kann. Der Elektrolyt dieses Sensors besteht aus bzw. enthält Sulfide. Diese Anordnung führt zu sehr stabilen Sensorsignalen, die elektronisch gut nachverstärkt werden können. Ein erheblicher Nachteil bei dieser Anordnung ist jedoch, dass der Sensor nicht in Umgebungen eingesetzt werden kann, die saure Gase, wie z.B. Kohlendioxid, enthalten. In diesem Fall würde giftiger Schwefelwasserstoff freigesetzt. Eine Anwendung in der Medizintechnik verbietet sich daher.

In der Patentschrift GB 1 391 168 wird eine Vorrichtung zur Messung von Sauerstoff beschrieben. Der besondere konstruktive Aufbau mit zwei sauerstoffdurchlässigen Membranen, von denen eine porös ist, einer Kathode aus Silber und einer rohrförmigen Zinnanode erlaubt die Messung von Sauerstoff in kondensierenden Medien oder in Umgebungen die Wassertröpfchen enthalten, wie z.B. Atemgasbefeuchtern. Die poröse Membran verhindert auf Grund ihres hydrophoben Charakters die Ausbildung eines geschlossenen Wasserfilms auf der Oberfläche, der zu einer Signalreduktion führen kann. Über die Zusammensetzung des Elektrolyts werden keine Angaben getroffen. Die Technik der Kombination zweier sauerstoffdurchlässiger Membranen, von denen eine porös ist und dem Schutz vor Wasserkondensation dient, hat sich bei der Konstruktion von Sauerstoffsensoren als Standard durchgesetzt.

In dem Patent US 4,664,119 wird ein Sauerstoffsensor bestehend aus einem Gehäuse, einer Kathode, einer Diffusionsbarriere, Kontaktdrähten und einer aus einer zinnhaltigen Legierung bestehenden Anode beschrieben. Als mögliche Elektrolyte werden, Essigsäure, Weinsäure, Zitronensäure, Apfelsäure, Oxalsäure und Salzsäure vorgeschlagen. Dieser Sensor ist für die transkutane Messung von Sauerstoff konzipiert.

In EP 1 593 962 wird ein RoHS konformer, galvanischer Sauertsoffsensor beschrieben, dessen Anode aus Zink oder Aluminium besteht. In der Anmeldung wird beschrieben, dass diese Materialien jedoch korrosionsanfällig und nur innerhalb relativ enger Elektrolyt pH-Werte stabil sind. Zudem steigt bekanntlich der Korrosionsstrom mit der Temperatur steil an, sodass derartige Sensoren bei Anwendungen in höheren Temperaturbereichen eine stark eingeschränkte Lebensdauer aufweisen. Weiterhin wird durch den Korrosionsvorgang Wasserstoff gebildet, der aus dem Sensor abgeführt werden muss. Dies erfordert eine aufwändige Konstruktion des Sensorgehäuses. Weiterhin zeigen solche Sensoren eine vergleichsweise hohe Ansprechzeit.

Einen RoHS konformen galvanischen Sauerstoffsensor mit geringerer Ansprechzeit beschreibt US 2010/0252432. Der Sensor verfügt vorzugsweise über eine Zinnanode. Dem Elektrolyten dieses Sensors ist ein Chelatbildner z.B. EDTA zugesetzt. Der Chelatbildner soll die Ablagerung von Reaktionsprodukten an der Anode verhindern, die wiederum zu einer Passivierung der Anodenoberfläche führen können. Der Elektrolyt ist stark alkalisch mit einem pH-Wert vorzugsweise über 12. Als Kathodenmaterial kommt neben Silber oder Platin vorzugsweise Gold zum Einsatz. Das Gold ist auf die Diffusionsmembran des Sensors aufgesputtert. Tests haben gezeigt, dass Konzentrationen von ca. 1 mol/L eingesetzt werden müssen, um die Sensoreigenschaften über mehrere Monate stabil zu erhalten. Ob diese Sensoren eine übliche Mindestlebenszeit von 2 Jahren erreichen ist fraglich. Weiterhin fraglich ist, ob der Elektrolyt sich über die Lebenszeit des Sensors nicht entmischt. Des weiteren weisen solche Sensoren eine hohe Querempfindlichkeit gegenüber CO₂ und Lachgas (N₂O) auf. Daher sind sie für einen Einsatz insbesondere in der Medizintechnik nicht geeignet.

Die Querempfindlichkeit stellt die Empfindlichkeit einer Messeinrichtung auf andere Größen als die Messgröße dar; letztere ist die zu messende Größe. Eine Größe, die nicht Messgröße ist, jedoch die von der Messeinrichtung gelieferte Information über den Messwert beeinflusst, heißt Einflussgröße. Sie bewirkt, dass sich der Messwert allein schon dadurch ändert, dass sich die Einflussgröße ändert. Zu den Zielen einer jeden Messgeräteentwicklung soll gehören, Querempfindlichkeit gering zu halten. Zu einer Querempfindlichkeit trägt auch unvollständige Selektivität bei, wie sie z.B. in Gassensoren vorkommt. Diese sprechen oft auch auf Konzentrationen anderer Gase an als des zu detektierenden Gases.

Einen weiteren RoHS konformen galvanischen Sauerstoffsensor beschreibt die WO 2013/039414. Der Sensor wird mittels einer Kapillare, die den Gaszutritt begrenzt als sogenannter ,Limiting current' Sensor betrieben. Er nutzt alternativ einen alkalischen oder sauren Elektrolyten, eine Kathode entweder aus Platin/Kohlenstoff oder aus Silber oder Gold und eine Antimon-, Bismut- oder Kupferanode. Ein Nachteil dieser Materialpaarungen ist, dass unlösliche Reaktionsprodukte anfallen, die die reaktiven Oberflächen nicht blockieren dürfen. Dies konstruktiv sicherzustellen erfordert einen hohen Aufwand. Ein weiterer Nachteil beim Einsatz von saurem Elektrolyt ist, dass aufgrund des Protonenverbrauchs der chemischen Reaktionen ein Elektrolytvolumen von ca. 20 mL erforderlich ist, um eine Lebenszeit von 2 Jahren zu gewährleisten. Solch ein Sensor ist aufgrund der größeren Baugröße nicht rückwärtskompatibel zu herkömmlichen galvanischen Sensoren mit Bleianode.

Die Anmeldung WO 2013/049752 betrifft einen Sensor mit reiner Bismuthanode, saurem Elektrolyt und einer gesputterten Goldkathode.

Die DE 10 2006 024 022 A1, von der Anmelderin der vorliegenden Erfindung, betrifft einen RoHS konformen galvanischen Gassensor bestehend aus einem Gehäuse, einer zinnhaltigen Anode, einer Diffusionsbarriere, einer Kathode und einem Elektrolyten, wobei der Elektrolyt vorzugsweise aus einer wässrigen Lösung von Cäsiumsalzen besteht oder auch aus einer phosphorsauren Lösung.. Gemäß einer bevorzugten Ausfuhrungsform kann die zinnhaltige Anode zudem Silber und/oder Kupfer aufweisen. Aufgrund der geringen Sensorströme verfügen solche Sensoren über eine ausgesprochen lange Lebensdauer von 4 bis 5 Jahren. Sie können mit der entsprechenden integrierten elektronischen Schaltung rückwärtskompatibel zu klassischen galvanischen Sauerstoffsensoren angeboten werden. Weiterhin zeichnen sie sich durch eine sehr niedrige CO₂-Querempfindlichkeit aus und haben sich daher in verschiedenen industriellen Anwendungen seit Jahren bewährt. Allerdings zeigen solche Sensoren teilweise eine deutliche Querempfindlichkeit gegenüber Lachgas und sind daher für spezielle medizinische und andere Anwendungen, bei denen Lachgas im Messgasgemisch vorkommt, nur bedingt geeignet.

Lachgas wird in der Medizintechnik zu Narkosezwecken benutzt und dem Patienten im Atemgasgemisch zugeführt. Die Sauerstoffkonzentration in solchen Narkosegasgemischen wird häufig mit Hilfe von elektrochemischen Sensoren gemessen. Lachgas ist weiterhin auch in verschiedenen industriellen Gasgemischen enthalten, beispielsweise in der Lebensmittelindustrie und Abgasmessung an Verbrennungsmotoren.

Es wird vermutet, dass die Lachgasquempfindlichkeit dieses Sensors auf der Verunreinigung der kupferhaltigen Kathode durch Spuren von Silber beruht. In C.E.W. Hahn, Analyst, 1998, 123, 57R - 86R wird beschrieben, das Lachgas an silberhaltigen Oberflächen elektrochemisch reduziert wird. Dies hat bereits in den 1970er Jahren zu Fehlfunktionen bei Gas- und Blutgassensoren geführt. Eigene Experimente zeigen, das bereits geringste Spuren von Silber, die selbst in hochreinem Kupfer enthalten sind zu Querempfindlichkeiten von drei bis zehn Prozent führen können. Dies verbietet die medizinische Anwendung solcher Sensoren, da in diesen Anwendungen die Querempfindlichkeit des Sensors gegen ein anderes Gas gemäß der Norm ISO 80601-2-55 maximal 0,3 % des angezeigten Sauerstoffwertes betragen darf.

Mit der Ausdehnung der RoHS-Gesetzgebung auch auf medizinische Geräte und Kontroll- und Überwachungsinstrumente ab Mitte 2014 ist es erstrebenswert, möglichst rückwärtskompatible galvanische Sauerstoffsensoren zur Verfügung zu stellen. Diese Sensoren sollten insbesondere gemäß der Norm ISO 80601-2-55 nur minimale Querempfindlichkeiten gegenüber Lachgas aufweisen. Dies gelingt bisher nicht mit den zuvor beschriebenen RoHS konformen galvanischen Sauerstoffsensoren.

Aufgabe der Erfindung ist es daher, einen neuartigen galvanischen Sauerstoffsensor bereitzustellen, der RoHS konform ist und in seinen sonstigen elektrischen und geometrischen Spezifikation und auch seiner Lebenszeit vorzugsweise rückwärtskompatibel zu den bestehenden bleihaltigen Sensoren ist und vorzugsweise keine Querempfindlichkeit gegenüber Lachgas aufweist. Solche Sensoren können nicht nur bei der Erstinstallation eingesetzt werden, sondern auch als Ersatzteile für die vielen im Markt befindlichen Instrumente und Geräte genutzt werden.

### ZUSAMMENFASSUNG DER ERFINDUNG

Erfindungsgemäß wird die Aufgabe durch die Merkmale des unabhängigen Anspruchs 1 gelöst. Vorteilhafte Ausgestaltungen ergeben sich aus den Unteransprüchen.

Voraussetzung für die Funktion eines elektrochemischen Sauerstoffsensors ist, dass ein Großteil, vorzugsweise sämtliche an die Kathode diffundierenden Sauerstoffmoleküle elektrochemisch reduziert werden. Dies gelingt jedoch nur dann zuverlässig, wenn die Anode genügend elektropositiv ist, d.h. genügend Elektronen für die Sauerstoffreduktion zur Verfügung stellt. Andererseits darf das Anodenmaterial auch nicht zu elektropositiv sein, da dann an der Kathode Wasserstoff entwickelt wird. In diesem Fall würde sich der Sensor rasch selbst verbrauchen, zudem wäre ein Grundstrom vorhanden, der sich dem eigentlichen Signal überlagert. Die Wasserstoffentwicklung kann zwar durch die Elektrolytzusammensetzung und/oder Zusammensetzung bzw. Beschichtungen an der Kathode etwas beeinflusst werden, jedoch nur in geringerem Umfang.

Verschärft wird die Situation noch durch die Temperaturabhängigkeit der elektrochemischen Potentiale. Der typische Arbeitsbereich bzw. zugelassene Temperaturbereich der Sensoren liegt zwischen -20° und 60°C, umfasst also ca. 80 Kelvin Differenz, in denen der Arbeitspunkt der Anode stabil liegen muss. Ansonsten kann entweder eine Wasserstoffentwicklung oder eine unvollständige Umsetzung des Sauerstoffs an der Kathode erfolgen, mit einer Nichtlinearität des Sauerstoffsignals als Konsequenz.

Ein weiteres Risiko betrifft die Korrosionsfestigkeit des Anodenmaterials im Elektrolyten innerhalb des spezifizierten Temperaturbereiches. Die Lebensdauer von Sensoren herkömmlicher Bauart liegt derzeit bei 2-3 Jahren und die neuen RoHS konformenSensoren sollten vorzugsweise zumindest die gleiche Lebenszeit aufweisen. Diese Lebensdauer kann beispielsweise erreicht werden, wenn das Metall an der Oberfläche entweder durch eine Oxidschicht und/oder durch Bildung eines schwerlöslichen Salzes geschützt wird. Dabei sollte die Schicht vorzugsweise über die gesamte Lebensdauer des Sensors leitfähig bleiben, da ansonsten die elektrochemische Reaktion eingeschränkt oder gestoppt wird.

Auch die Umsetzung der Idee eines "einstellbaren" elektrochemischen Potentials über die Legierungszusammensetzung birgt Risiken, da Legierungen häufig zu intermetallischen Phasen und Ausbildung von Eutektika neigen. Diese wiederum haben meist andere Eigenschaften als die homogenen Mischphasen.

Für einen galvanischen Sauerstoffsensor mit Elektrolyt und Anode, lauten die Gleichungen der elektrochemischen Reaktionen wie folgt:
Gleichung für den elektrochemischen Vorgang an der Kathode in einem alkalischen Elektrolyt:

O₂ + 2H₂O + 4e⁻→ 4OH⁻ (Gl. 1a)

In einem sauren Elektrolyt verläuft die Reduktion des Sauerstoffs unter Verbrauch von Protonen:

O₂ + 4H⁺ + 4e⁻→2H₂O (Gl.1b)

Gleichungen für mögliche, vom lokalen pH-Wert an der Anodenoberfläche abhängige elektrochemische Vorgänge an der Anode:

Me + 4OH⁻→ MeO₂ + 4e⁻ + 2H₂O (Gl. 2a)

2Me → 2Me²⁺ + 4e⁻ (Gl. 2b)

Me steht dabei stellvertretend für ein Metall, das nach der Oxidation in 2-wertiger oder einer 4-wertiger Oxidationsstufe auftreten kann. Nach Gl. 2a erfolgt die Oxidation des Anodenmaterials zu einem Oxid, nach Gl. 2b entsprechend zu einem löslichen Salz.

Der über die galvanische Zelle fließende elektrische Strom ist vorzugsweise linear oder nahezu linear vom Sauerstoffpartialdruck bzw. der Sauerstoffkonzentration abhängig. Die elektrochemisch aktive Fläche der Anode sollte vorzugsweise größer als die der Kathode sein, um eine ausreichende Triebkraft der Reaktion sicher zu stellen und einer Konzentrationspolarisation an der Oberfläche der Anode zu vermeiden.

Aufgrund des speziellen Charakters der Aufgabenstellung lassen sich Informationen über geeignete Materialpaarungen leider nur sehr eingeschränkt aus der Literatur gewinnen. Die erforderlichen elektrochemischen Eigenschaften können nur näherungsweise berechnet werden und müssen daher experimentell ermittelt werden.

Überraschenderweise zeigen Zweistofflegierungen aus Zinn und Silber oder Kupfer und insbesondere auch Dreistofflegierungen aus Zinn, Silber und Kupfer hervorragende Eigenschaften sowohl in sauren als auch alkalischen Elektrolyten. Zinn als reines Metall führt in sauren Elektrolyten zu Wasserstoffentwicklung an der Kathode und ist somit nur mit alkalischen Elektrolyten einsetzbar. Silber- und Kupferanteile in einer Legierung mit Zinn vermindern jedoch schon in geringen Anteilen das elektropositive Verhalten des Zinns erheblich. Es hat sich gezeigt, dass bereits Anteile von 0,1-3 % Silber und/oder Kupfer ausreichen, um die Wasserstoffentwicklung über den gesamten Temperaturbereich genügend einzuschränken. Auch höhere Anteile von Silber oder Kupfer in der Legierung sind möglich, im Hinblick auf die Ausbeute des Materials und auch unter Betracht von Herstellkosten sind sie jedoch weniger interessant.

Die Legierungen sind über den nötigen Temperaturbereich weitgehend korrosionsfest und liefern ein ausreichendes elektrochemisches Potential, um Sauerstoff an der Kathode zu reduzieren. Dies gelingt vorzugsweise besonders gut, wenn als Kathodenmaterial Kupfer zum Einsatz kommt.

Wünscht man kleinere Sensorströme genügt als Kathode im einfachsten Fall schon ein Draht oder ein dünnes Band, vorzugsweise aus Kupfer, welches sich in Kontakt mit dem Elektrolyt befindet, direkt hinter der Diffusionsmembran liegt und mit einem Kontaktdraht versehen ist, der beispielsweise durch das Gehäuse nach außen führt. Der Kontaktdraht kann beispielsweise im Gehäuse verklebt, mittels Ultraschall oder thermisch mit dem Kunststoff verschweißt werden, um Elektrolytaustritt aus dem Gehäuse zu vermeiden. Vorteilhaft gestaltet man die Führung des Kontaktdrahtes so, dass möglichst wenig seiner Oberfläche in Kontakt mit Elektrolyt steht. Da im Elektrolyt geringe Mengen Sauerstoff gelöst sind, könnte diese Fläche zum Grundstrom des Sensors beitragen und das Sensorverhalten in Umgebungen mit geringen Sauerstoffgehalten ungünstig beeinflussen.

Besonders kleine Sensorströme erlauben eine einfache Auslegung der Anodenkontaktierung bei gleichzeitiger Erhöhung der Lebenszeit des Sensors. Dies ermöglicht prinzipiell sehr kleine Bauformen der Sensoren, für den Fall, dass keine Rückwärtskompatibilität zu existierenden Sensoren gefordert ist.

Auch die Zusammensetzung des Elektrolyts beeinflusst die Funktion des Sensors. In Kombination mit den beschriebenen Anodenmaterialien werden die besten Ergebnisse mit wässrigen alkalischen Lösungen aus Alkali- oder Erdalkalimetallsalzen erzielt. Neben den guten elektrochemischen Eigenschaften weisen diese Lösungen auch ein leicht hygroskopisches Verhalten auf, das einem Eintrockenen des Elektrolyten bei möglichen trockenen Arbeitsbedingungen des Sensors entgegenwirkt und damit zu einer langen Lebensdauer des Sensors auch unter rauen Bedingungen beiträgt.

Sehr gute Ergebnisse erzielt man beispielsweise mit wässrigen Lösungen von Cäsiumsalzen, insbesondere Cäsiumhydroxid, Cäsiumcarbonat, Cäsiumhydrogencarbonat und Cäsiumacetat, oder Mischungen derselben. Diese Salze lösen sich sehr gut in Wasser, sind teilweise hygroskopisch und tragen damit zu einer langen Standzeit des Sensors bei. Durch die gute Löslichkeit wird bei höheren Konzentrationen der Anteil von im Elektrolyt gelöstem Sauerstoff reduziert und damit eine schnelle Ansprechzeit und ein sauberes Grundstromverhalten des Sensors erreicht. Vergleichbare Ergebnisse können auch durch den Einsatz von Magnesium-, Natrium- oder Kaliumsalzen erzielt werden.

Um die unerwünschte Querempfindlichkeit eines solchen Sensors gegen Lachgas auf ein akzeptables Maß zu reduzieren, muss die katalytische Aktivität der Silberverunreinigungen in der Kupferkathode blockiert werden. Hierfür können gezielt Katalysatorgifte dem Elektrolyt und/oder der Kathode zugesetzt werden. Insbesondere ist es gemäß bestimmter Ausführungsformen auch möglich, wenn das Katalysatorgift in einem extra Bauteil bzw. in einem extra Fluid im Sensors bereitgestellt wird, solange das Katalysatorgift mit dem Elektrolyt bzw. der Kathode in Verbindung steht. Zudem ist es auch möglich reinen Schwefel oder eine Schwefelverbindung im Sensor als "Katalysatorgiftspender" bereitzustellen. Mit anderen Worten, das Katalysatorgift wird in dem Sensor so bereitgestellt, dass es (vorzugsweise während einer chemischen Reaktion) zur Kathode gelangen kann, vorzugsweise über den Elektrolyt zur Kathode gelangen kann. Katalysatorgifte binden sich chemisch an die aktiven Zentren des Katalysators und blockieren diese.

Als Katalysatorgift kommen grundsätzlich verschiedene Substanzen wie z.B. Schwefel oder schwefelhaltige Verbindungen, Blei- und Bleiverbindungen, Chinoline, Pyridine, Halogenide oder Kohlenmonoxid in Frage. Die vorliegende Erfindung ist jedoch nicht auf die Verwendung genau eines Katalysatorgifts beschränkt. So können beispielsweise ein, zwei oder mehr Katalysatorgifte im Sensor verwendet werden. Unter Katalysatorgift, bei heterogenen Katalysatoren auch Kontaktgift, versteht man einen Stoff, der die Wirkung eines Katalysators dauerhaft verringert oder aufhebt. Dadurch bremst es eine, üblicherweise erwünschte, chemische Reaktion.

Chemische Katalysatoren können durch eine Reihe von Stoffen beeinträchtigt oder zerstört werden, darunter Schwermetalle, Halogene, Polymere, Schwefel oder Kohlenstoffmonoxid. Katalysatorgifte werden vom Katalysator statt des Stoffes, dessen Reaktion er beschleunigen soll, gebunden. Sie blockieren dadurch die Adsorptionsfähigkeit der großen Oberfläche der Katalysatorpartikel. Die Vergiftung eines Katalysators kann erwünscht sein, um die Aktivität eines Katalysators für eine spezielle Reaktion gezielt zu verringern. Oxidationskatalysatoren werden gezielt vergiftet um etwa die Oxidation von primären Alkoholen zu Aldehyden zu steuern und nicht zu Carbonsäuren zu oxidieren. Durch die gezielte Vergiftung von Reduktionskatalysatoren mit Schwefelverbindungen kann man zum Beispiel erreichen, dass Alkine zu Alkenen, nicht aber weiter zu Alkanen hydriert werden.

Die Wirkung des erfindungsgemäßen Katalysatorgiftes soll anhand des folgenden Beispiels näher erläutert werden.

Das kupferhaltige Kathodenmaterial kann in Spuren Verunreinigungen, z. B. Silber oder andere Metalle enthalten. Diese Metalle stellen Reaktionszentren dar, die die Reduktion von Lachgas (N₂O) an der Kathode katalysieren können:

N₂O + H₂O + 2e⁻ --> N₂ + 2OH⁻

Durch Zusatz schwefelhaltiger Verbindungen, z. B. Natriumthiosulfat, reagieren die Verunreinigungen, z. B. Silber, mit der Schwefelspezies und bilden Sulfide. Die Reaktion wird nachstehend anhand von Silber und Natriumthiosulfat erläutert:

| | |
|---|---|
| Bildung von Silber-Ionen: | Cu₂O + 2Ag + H₂O → 2Ag⁺ + 2Cu + 2OH⁻ |
| Reaktion mit Natriumthiosulfat: | Na₂S₂O₃ + 2Ag⁺ → Ag₂S₂O₃ (instabil) + 2Na⁺ |
| Zerfall Silberthiosulfat: | Ag₂S₂O₃ + 2OH⁻ → Ag₂S + SO₄²⁻ + H₂O |

Durch die Sulfidbildung findet beispielsweise eine Inaktivierung der Reaktionszentren der Silberverunreinigungen statt. Somit kann die Reduktion von Lachgas an der Kathode unterdrückt werden. Aufgrund einer Oxidschicht auf der Kathode und dem unterschiedlichen Potential gegenüber der Silber-Verunreinigung findet diese Reaktion am Kupfer der Kathode nicht bzw. unterdrückt statt. D.h. die gewünschte Reduktion des zu messenden Sauerstoffs an der Kupferkathode läuft nahezu unbeeinträchtigt ab.

Insbesondere betrifft die vorliegende Erfindung einen galvanischen Sauerstoffsensor mit einem Gehäuse, einer Kathode, einer zinnhaltigen Anode, einer Diffusionsbarriere und einem wässrigen Elektrolyt mit Metallsalzen. Vorzugsweise ist dem Elektrolyt und/oder der Kathode mindestens ein Katalysatorgift zugesetzt. Alternativ oder zusätzlich kann der Sensor auch ein extra Bauteil bzw. ein extra Fluid mit Katalysatorgift aufweisen, wobei das Bauteil bzw. das Fluid vorzugsweise mit dem Elektrolyt bzw. der Kathode in Verbindung steht.

Das Katalysatorgift verringert bzw. verhindert vorzugsweise die Aufspaltung von Lachgas an der Kathode. Der erfindungsgemäße Sauerstoffsensor dient beispielsweise zur Anwendung in der Medizintechnik, insbesondere zur Anwendung in Anästhesiemaschinen, Inkubatoren und/oder Beatmungsgeräten. Der erfindungsgemäße Sauerstoffsensor kann jedoch auch für industrielle Gasmesstechnik verwendet werden, insbesondere zur Anwendung in der Emissionsmessung und/oder Lebensmitteltechnologie.

Der galvanische Sauerstoffsensor hat vorzugsweise eine Querempfindlichkeit gegenüber Lachgas von maximal 0,3 % des angezeigten Sauerstoffwertes.

Gemäß einer bevorzugten Ausführungsform handelt es sich bei dem Katalysatorgift um eine schwefelhaltige Verbindung, insbesondere um (reinen) Schwefel oder einer oder mehrerer schwefelhaltigen Verbindungen wie Thiosulfat und/oder Polysulfid. Vorzugsweise beträgt der Schwefel, Thiosulfat- und/oder Polysulfidanteil im Elektrolyt und/oder in der Kathode zwischen 0,0001 und 10%. Insbesondere kann der Thiosulfat- und/oder Polysulfidanteil im Elektrolyt und/oder der Kathode zwischen 0,0001 und 10 % betragen, vorzugsweise im Bereich 0,01-10%, 0,1 - 10%, 1-10%, 0,0001 -5%, 0,0001-2% bzw. 0,0001-1%.

Vorzugsweise beträgt der Schwefel, Thiosulfat- und/oder Polysulfidanteil im Bauteil zwischen 0,0001 und 30%. Insbesondere kann der Thiosulfat- und/oder Polysulfidanteil im Bauteil zwischen 0,0001 und 30 % betragen, vorzugsweise im Bereich 0,01-30%, 0,1 - 30%, 1-30%, 0,0001 -15%, 0,0001-10%, 0,0001 -5%, 0,0001-2% bzw. 0,0001-1%.

Vorzugsweise handelt es sich bei dem Katalysatorgift um Blei- oder eine Bleiverbindungen, wobei der Bleianteil des Elektrolyten, des Bauteils und/oder der Kathode vorzugsweise maximal 0,1 % beträgt.

Vorzugsweise kann es sich beim dem Katalysatorgift (4) um Chinolin und/oder Halogenid und/oder Kohlenmonoxid und/oder Pyridin handeln.

Vorzugsweise weist der Elektrolyt einen pH-Wert größer 7 auf.

Vorzugsweise handelt es sich bei den Metallsalzen im Elektrolyt um Alkali- oder Erdalkalimetallsalze, insbesondere um Magnesium-, Natrium-, Kalium- oder Cäsiumcarbonate, - hydrogencarbonate und um Salze ihrer organischen Säuren oder um eine Mischung dieser Substanzen.

Gemäß einer weiteren bevorzugten Ausführungsform weist die Kathode Kupfer oder ein verkupfertes Bauteil oder eine Kupferlegierung auf.

Gemäß einer weiteren bevorzugten Ausführungsform weist das Anodenmaterial Zinn oder Legierungen des Zinns mit Silber und/oder Kupfer auf, wobei der Gesamtanteil von Silber und Kupfer vorzugsweise mindestens 0,1% und höchstens 25 % der Gesamtmasse beträgt, der Kupferanteil vorzugsweise zwischen 0,1 - 2%, 0,5 - 5%, 2 - 15% oder 5 - 25% beträgt und der Silberanteil vorzugsweise zwischen 0,1 - 2%, 0,5 - 5%, 2 - 15% oder 5 - 25% beträgt.

Gemäß einer weiteren bevorzugten Ausführungsform weist der Sauerstoffsensor einen Kontaktdraht an der Anode auf der Nickel enthält.

Zudem betrifft die vorliegende Erfindung auch Verfahren zur Messung von Sauerstoff mit einem erfindungsgemäßen galvanischer Sauerstoffsensor. Hierzu wird eine zusätzliche Auswertelektronik verwendet, mit der Signale vom Sauerstoffsensor ausgewertet werden und in einen Sauerstoffgehalt umgerechnet werden.

Neben der Anwendung des zuvor beschriebenen galvanischen Sensors als Sauerstoffsensor ist es jedoch auch möglich, die Querempfindlichkeit gegenüber Lachgas, die der Sensor ohne den Zusatz eines Katalysatorgifts aufweist, zur gezielten Messung von Lachgas zu nutzen. Vorzugsweise sollte eine verbleibende Querempfindlichkeit gegenüber Sauerstoff durch geeignete Maßnahmen kompensiert werden. Beispielsweise kann eine 2. Kathode mit geringer Sauerstoffquerempfindlich im selben Elektrolyt platziert werden und deren Signal mit dem der 1. Kathode verrechnet werden. Eine weitere Möglichkeit besteht darin, die 2. Kathode in einem mechanisch abgegrenzten 2. Elektrolytraum zu platzieren. In diesem Fall ist der Einsatz zweier unterschiedlicher Elektrolyte möglich, um die Selektivität der beiden Kathodenreaktionen unterschiedlich zu steuern. Auch in diesem Fall erfolgt vorzugsweise eine Verrechnung der beiden Sensorsignale, um die jeweilige Restquerempfindlichkeit zu kompensieren.

Derart hergestellte Sauerstoffsensoren zeigen über einen weiten Temperaturbereich ein stabiles und vorzugsweise lineares Signalverhalten gegenüber dem Sauerstoffpartialdruck. Über die Gestaltung der Kathodenfläche kann der Sensorstrom so gesteuert werden,dass er in der gleichen Größenordnung wie die bisherigen, mit Bleianoden aufgebauten Sensoren liegt. Eine Rückwärtskompatibilität zu den im Markt befindlichen Geräten ist damit gegeben.

### KURZE BESCHREIBUNG DER ZEICHNUNGEN

Im Folgenden werden bevorzugte Ausführungsformen der vorliegenden Erfindung unter Bezugnahme auf die Figuren ausführlich beschrieben. Es zeigen:
- Fig. 1: eine Querschnittsansicht eines erfindungsgemäßen galvanischem Sauerstoffsensors;
- Fig. 2: die Abhängigkeit eines Sensorsignals vom Sauerstoffpartialdruck;
- Fig. 3: das Signalverhalten eines erfindungsgemäßen Sensors bei der Begasung mit 100% Lachgas; und
- Fig. 4: das Verhalten eines erfindungsgemäßen Sensors bei der Begasung mit einer Gasmischung aus 45 % Sauerstoff, 5 % Kohlendioxid, 48,5 % Lachgas und 1,5 % Isofluran.

### DETAILLIERTE BESCHREIBUNG BEVORZUGTER AUSFÜHRUNGSFORMEN

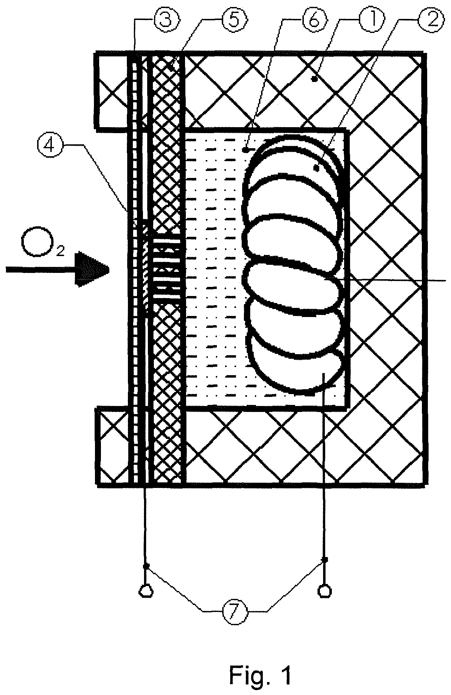

Ein galvanischer, elektrochemischer Sauerstoffsensor entsprechend Fig. 1 umfasst als wesentliche Komponenten vorzugsweise ein Gehäuse 1, Diffusionsbarriere 3, eine Anode 2 sowie eine Kathode 4. Die Anode 2 ist vorzugsweise im Gehäuse 1 angeordnet und beispielsweise aus einer Legierung von Zinn mit 3 % Kupfer hergestellt, mit einem bevorzugten Gewicht von 2,5 Gramm. Die Diffusionsbarriere 3 bildet eine Grenzschicht zur messenden Gasumgebung, d.h. nach außen und ist beispielhaft als Diffusionsmembran 3 aus Polytetrafluorethylen hergestellt. Die Kathode 4, vorzugsweise aus einem Flachdraht aus Kupfer, ist beispielsweise hinter der Diffusionsmembran 3 auf einer Unterlage 5 angeordnet. Ein Elektrolyt 6 ist vorzugsweise zwischen der Kathode 4 und der Anode 2 angeordnet, vorzugsweise zwischen der Unterlage 5 und der Anode 2. Vorzugsweise umgibt der Elektrolyt 6 einen Großteil der Anode 2. Der Elektrolyt 6 ist beispielsweise eine wässrige Lösung mit 20 % Cäsiumcarbonat, 10 % Cäsiumhydrogencarbonat und 1 g/L Natriumthiosulfat mit einem pH-Wert von ca. 10. Die Kontaktierungen 7, die mit der Anode bzw. Kathode verbunden sind, werden vorzugsweise nach außen geführt, um für einen elektrischen Stromfluss zu sorgen, der gleichzeitig die Messgröße darstellt. Der über den Widerstand fließende elektronische Strom ist vorzugsweise dem an der Diffusionsmembran anstehenden Sauerstoffpartialdruck proportional oder annähernd proportional. Die Kathode 4 weist beispielsweise eine Fläche von 2,5 mm² auf. Mit einer 25 µm dicken Diffusionsmembran 3 wird an Luft ein Sensorsignal von 3 µA erhalten. Bei Begasung mit Stickstoff (N₂) fällt das Signal auf etwa 7,5 nA ab. Bei Begasung mit Sauerstoff (O₂) wird annähernd der theoretische Wert von 14,3 µA erreicht.

Der durch die Diffusionsmembran 3 diffundierende Sauerstoff gelangt an die Kathode 4, wo er reduziert wird. An der Anode 2 gehen entsprechend dem Faraday'schen Gesetz Metallionen in Lösung bzw. das Metall wandelt sich in Metalldioxid. In der in Fig. 1 dargestellten bevorzugten Ausführungsform ist das erfindungsgemäße Katalysatorgift dem Elektrolyt 6 zugesetzt. Die vorliegende Erfindung ist jedoch nicht auf diese Ausführungsform beschränkt. So kann alternativ oder zusätzlich Katalysatorgift der Kathode 4 zugesetzt sein. Gemäß einer weiteren nicht dargestellten Ausführungsform kann Katalysatorgift alternativ oder zusätzlich in einem extra Fluid und/oder einem extra Bauteil, vorzugsweise innerhalb des Sensors, bereitgestellt werden, um die Aufspaltung von Lachgas an der Kathode 4 zu verhindern oder zu verringern. Vorzugsweise ist das extra Bauteil oder extra Fluid in Verbindung mit dem Elektrolyt 6 und/oder der Kathode 4.

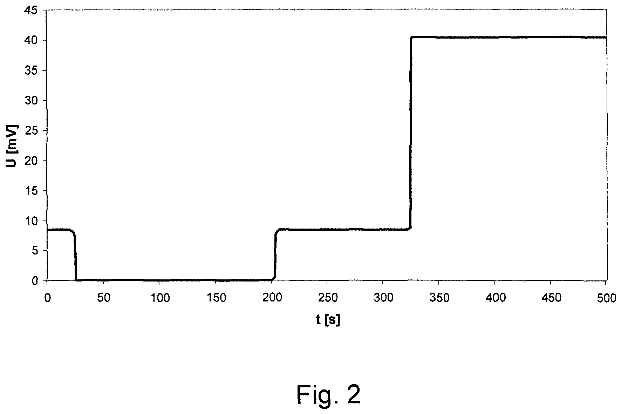

Wie aus Fig. 2 ersichtlich ist, zeigt ein derartig hergestellter Sauerstoffsensor eine ausgezeichnete Linearität zum Sauerstoffpartialdruck. Der Signalverlauf zeigt die Spannung des Sensors bei Begasung mit Luft, mit Stickstoff, mit Luft und mit 100% Sauerstoff. Das Sauerstoffsignal ist ca. 4,8 mal größer als das Luftsignal und entspricht damit dem theoretischen Wert.

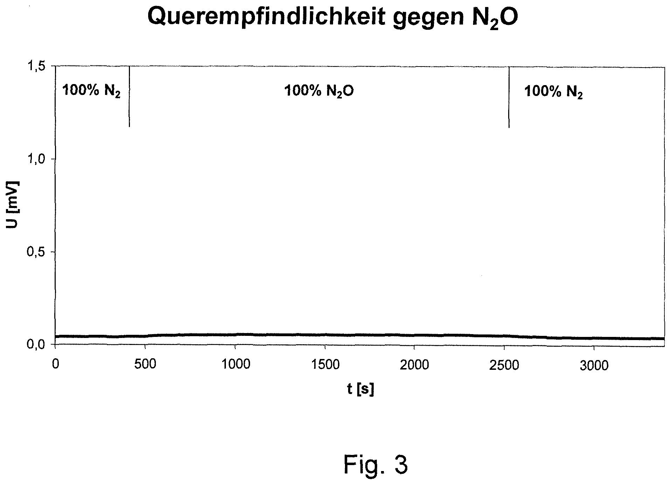

Aus Fig. 3 ist ersichtlich, dass ein derartig hergestellter Sauerstoffsensor keine nennenswerte Querempfindlichkeit gegenüber Lachgas aufweist. Der Signalverlauf zeigt die Spannung des Sensors nacheinander bei Begasung mit Stickstoff, mit Lachgas und mit Stickstoff.

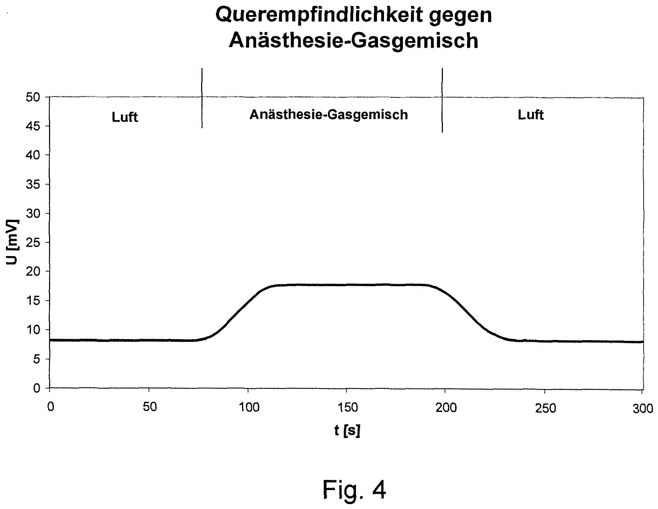

Auch bei der Messung in einem Anästhesiegasgemisch aus 48,5 % Lachgas, 45 % Sauerstoff, 5 % Kohlendioxid und 1,5 % Isofluran zeigt der Sensor keine nennenswerte Querempfindlichkeit, wenn man den Mischfehler des Prüfgases berücksichtigt. Der Signalverlauf in Fig. 4 zeigt die Spannung des Sensors nacheinander bei Begasung mit Luft, dem Anästhesiegasgemisch und mit Luft.

Die Erfindung umfasst ebenfalls die genauen oder exakten Ausdrücke, Merkmale, numerischen Werte oder Bereiche usw., wenn vorstehend oder nachfolgend diese Ausdrücke, Merkmale, numerischen Werte oder Bereiche im Zusammenhang mit Ausdrücken wie z.B. "etwa, ca., um, im Wesentlichen, im Allgemeinen, zumindest, mindestens" usw. genannt wurden (also "etwa 3" soll ebenfalls "3" oder "im Wesentlichen radial" soll auch "radial" umfassen). Der Ausdruck "bzw." bedeutet überdies "und/oder".

## Patentansprüche

1. Galvanischer Sauerstoffsensor mit:
einem Gehäuse (1), einer kupferhaltigen Kathode (4), einer zinnhaltigen Anode (2), einer Diffusionsbarriere (3) und einem wässrigen Elektrolyt (6) mit Metallsalzen,
**dadurch gekennzeichnet, dass** der Sauerstoffsensor mindestens ein Katalysatorgift aufweist,
das die Aufspaltung von Lachgas an der Kathode (4) verhindert oder verringert,
wobei es sich bei dem mindestens einen Katalysatorgift um Schwefel und/oder eine schwefelhaltige Verbindung in Form von Thiosulfat und/oder Polysulfid handelt, wobei der Schwefel-, Thiosulfat- und/oder Polysulfidanteil im Elektrolyt (6) und/oder der Kathode zwischen 0,0001 und 10% beträgt.

2. Galvanischer Sauerstoffsensor nach Anspruch 1, wobei der Elektrolyt (6) und/oder ein Bauteil/Fluid das mit dem Elektrolyt (6) in Verbindung steht und/oder die Kathode (4) mindestens ein Katalysatorgift aufweist.

3. Galvanischer Sauerstoffsensor nach einem der vorhergehenden Ansprüche 1 bis 2, dessen Querempfindlichkeit gegenüber Lachgas maximal 0,3 % des angezeigten Sauerstoffwertes beträgt.

4. Galvanischer Sauerstoffsensor nach einem der Ansprüche 1 bis 3, wobei der Schwefel, Thiosulfat- und/oder Polysulfidanteil im Bauteil zwischen 0,0001 und 30% beträgt.

5. Galvanischer Sauerstoffsensor nach einem der vorhergehenden Ansprüche, wobei zusätzlich das Katalysatorgift Blei- oder eine Bleiverbindungen vorhanden ist, wobei der Bleianteil des Elektrolyten maximal 0,1 % beträgt.

6. Galvanischer Sauerstoffsensor nach einem der vorhergehenden Ansprüche, wobei zusätzlich ein Katalysatorgift in Form von
(i) Chinolin und/oder
(ii) Halogenid und/oder
(iii) Kohlenmonoxid und/oder
(iv) Pyridin vorhanden ist.

7. Galvanischer Sauerstoffsensor nach einem der vorhergehenden Ansprüche, wobei der Elektrolyt (6) einen pH-Wert größer 7 aufweist.

8. Galvanischer Sauerstoffsensor nach einem der vorhergehenden Ansprüche, wobei es sich bei den Metallsalzen im Elektrolyt (6) um Alkali- oder Erdalkalimetallsalze handelt, insbesondere um Magnesium-, Natrium-, Kalium- oder Cäsiumcarbonate, -hydrogencarbonate und um Alkali- oder Erdalkalimetallsalze einer organischen Säure oder um eine Mischung dieser Substanzen handelt.

9. Galvanischer Sauerstoffsensor nach einem der vorhergehenden Ansprüche, wobei das Anodenmaterial Zinn oder Legierungen des Zinns mit Silber und/oder Kupfer aufweist, wobei der Gesamtanteil von Silber und Kupfer vorzugsweise mindestens 0,1% und höchstens 25 % der Gesamtmasse beträgt, der Kupferanteil vorzugsweise zwischen 0,1 - 2%, 0,5 - 5%, 2 - 15% oder 5 - 25% beträgt und der Silberanteil vorzugsweise zwischen 0,1 - 2%, 0,5 - 5%, 2 - 15% oder 5 - 25% beträgt.

10. Galvanischer Sauerstoffsensor nach einem der vorhergehenden Ansprüche, wobei der Sauerstoffsensor einen Kontaktdraht (7) an der Anode aufweist der Nickel enthält.

11. Verfahren zur Messung von Sauerstoff mit den Schritten:
Bereitstellen eines galvanischen Sauerstoffsensors nach einem der Ansprüche 1 bis 10;
Bereitstellen einer Auswertelektronik zum Auswerten von Signalen des Sauerstoffsensors,
Bestimmen des Sauerstoffgehalts mit Hilfe der Auswertelektronik.

## Claims

1. A galvanic oxygen sensor comprising:
a housing (1), a copper-containing cathode (4), a tin-containing anode (2), a diffusion barrier (3) and an aqueous electrolyte (6) with metal salts,
**characterized in that** the oxygen sensor comprises at least one catalyst poison which prevents or reduces the decomposition of nitrous oxide at the cathode (4),
wherein the at least one catalyst poison is sulfur and/or a sulfur-containing composition such as thiosulfate and/or polysulfide, wherein the sulfur, thiosulfate and/or polysulfide content in the electrolyte (6) and/or the cathode lies between 0.0001 and 10 %.

2. The galvanic oxygen sensor according to claim 1, wherein the electrolyte (6) and/or a component/fluid which is in connection with the electrolyte (6) and/or the cathode (4) comprises at least one catalyst poison.

3. The galvanic oxygen sensor according to claim 1 or 2 whose cross-sensitivity to nitrous oxide is at most 0.3 % of the indicated oxygen value.

4. The galvanic oxygen sensor according to any one of claims 1 to 3 wherein the sulfur, thiosulfate and/or polysulfide content in the component lies between 0.0001 and 30 %.

5. The galvanic oxygen sensor according to any one of the preceding claims, wherein additionally the catalyst poison is lead or a lead composition, wherein the lead content of the electrolyte is at most 0.1 %.

6. The galvanic oxygen sensor according to any one of the preceding claims, wherein additionally catalyst poison is present in the form of
(i) quinoline and/or
(ii) halogenide and/or
(iii) carbon monoxide and/or
(iv) pyridine.

7. The galvanic oxygen sensor according to any one of the preceding claims, wherein the electrolyte (6) has a pH value greater than 7.

8. The galvanic oxygen sensor according to any one of the preceding claims, wherein the metal salts in the electrolyte (6) are alkaline or alkaline earth metal salts, in particular magnesium, sodium, potassium or cesium carbonates or hydrogencarbonates and alkaline or alkaline earth metal salts of an organic acid or a mixture of these substances.

9. The galvanic oxygen sensor according to any one of the preceding claims, wherein the anode material comprises tin or tin alloys with silver and/or copper, wherein the total content of silver and copper is preferably at least 0.1 % and at most 25 % of the total mass, the copper content lies preferably between 0.1 to 2 %, 0.5 to 5 %, 2 to 15 % or 5 to 25 % and the silver content lies preferably between 0.1 to 2 %, 0.5 to 5 %, 2 to 15 % or 5 to 25 %.

10. The galvanic oxygen sensor according to any one of the preceding claims, wherein the oxygen sensor comprises a nickel-containing contact wire (7) at the anode.

11. A method for measuring oxygen comprising the steps:
providing a galvanic oxygen sensor according to any one of claims 1 to 10,
providing evaluation electronics for evaluating signals of the oxygen sensor,
determining the oxygen content by means of the evaluation electronics.

## Revendications

1. Capteur d'oxygène galvanique, comportant :
un boîtier (1), une cathode (4) contenant du cuivre, une anode (2) contenant de l'étain,
une barrière anti-diffusion (3) et un électrolyte (6) aqueux avec des sels métalliques,
**caractérisé en ce que** ledit capteur d'oxygène présente au moins un poison de catalyseur qui empêche ou réduit le fractionnement de protoxyde d'azote sur la cathode (4),
où ledit au moins un poison de catalyseur est du soufre et/ou un composé soufré sous forme de thiosulfate et/ou de polysulfure, la teneur en soufre, thiosulfate et/ou polysulfure de l'électrolyte (6) et/ou de la cathode étant comprise entre 0,0001 et 10%.

2. Capteur d'oxygène galvanique selon la revendication 1, où l'électrolyte (6) et/ou un composant/fluide en liaison avec l'électrolyte (6) et/ou la cathode (4) présentent au moins un poison de catalyseur.

3. Capteur d'oxygène galvanique selon l'une des revendications précédentes, dont la sensibilité croisée au protoxyde d'azote représente au maximum 0,3 % de la valeur d'oxygène indiquée.

4. Capteur d'oxygène galvanique selon l'une des revendications 1 à 3, où la teneur en soufre, thiosulfate et/ou polysulfure du composant est comprise entre 0,0001 et 30 %.

5. Capteur d'oxygène galvanique selon l'une des revendications précédentes, où en outre le poison de catalyseur plomb ou un composé de plomb est présent, la teneur en plomb de l'électrolyte étant de 0, 1 % au maximum.

6. Capteur d'oxygène galvanique selon l'une des revendications précédentes, où un poison de catalyseur est en outre présent sous forme
(i) de quinoléine et/ou
(ii) d'halogénure et/ou
(iii) de monoxyde de carbone et/ou
(iv) de pyridine.

7. Capteur d'oxygène galvanique selon l'une des revendications précédentes, où l'électrolyte (6) a un pH supérieur à 7.

8. Capteur d'oxygène galvanique selon l'une des revendications précédentes, où les sels métalliques de l'électrolyte (6) sont des sels métalliques alcalins ou alcalino-terreux, en particulier des carbonates de magnésium, de sodium, de potassium ou de césium, des carbonates d'hydrogène et des sels métalliques alcalins ou alcalino-terreux d'un acide organique, ou un mélange de ces substances.

9. Capteur d'oxygène galvanique selon l'une des revendications précédentes, où la matière d'anode comprend de l'étain ou des alliages d'étain avec de l'argent et/ou du cuivre, la teneur totale en argent et en cuivre étant préférentiellement comprise entre 0,1 % et 25 % de la masse totale, la teneur en cuivre préférentiellement comprise entre 0,1 et 2 %, 0,5 et 5 %, 2 et 15 % ou 5 et 25 % et la teneur en argent préférentiellement comprise entre 0,1 et 2 %, 0,5 et 5 %, 2 et 15 % ou 5 et 25 %.

10. Capteur d'oxygène galvanique selon l'une des revendications précédentes, où ledit capteur d'oxygène contient un fil de contact (7) sur l'anode, lequel contient du nickel.

11. Procédé de mesure d'oxygène, comprenant les étapes suivantes :
préparation d'un capteur d'oxygène galvanique selon l'une des revendications 1 à 10 ;
préparation d'une électronique d'analyse pour l'analyse des signaux du capteur d'oxygène,
détermination de la teneur en oxygène au moyen de l'électronique d'analyse.
